# EUROPEAN PATENT SPECIFICATION

(11) **EP 0 347 832 B1**
(45) Date of publication and mention of the grant of the patent: **28.06.1995**
(21) Application number: 89111181.7
(22) Date of filing: 20.06.1989
(51) Int. Cl.: A23B 7/005, A23L 1/222, A23L 1/235

(54) **Method for stabilizing taste-modifier**
Verfahren zum Stabilisieren eines Geschmacksveränderungsmittels
Procédé de stabilisation d'un agent modifiant le goût

(30) Priority: 21.06.1988 JP 153143/88; 02.11.1988 JP 277717/88; 11.11.1988 JP 285473/88
(43) Date of publication of application: 27.12.1989
(60) Divisional application: 93111742.8
(73) Proprietor: Kurihara, Yoshie, Tokyo 125 (JP); ASAHI DENKA KOGYO KABUSHIKI KAISHA, Arakawa-ku Tokyo 116 (JP)
(72) Inventor: Kurihara, Yoshie, Tokyo 125 (JP); Kohno, Hiroshige, Higashiogu 7-chome Arakawa-ku Tokyo 116 (JP); Kato, Masaaki, Higashiogu 7-chome Arakawa-ku Tokyo 116 (JP); Ikeda, Kenji, Higashiogu 7-chome Arakawa-ku Tokyo 116 (JP); Miyake, Masako, Higashiogu 7-chome Arakawa-ku Tokyo 116 (JP)
(74) Representative: Grünecker, Kinkeldey, Stockmair & Schwanhäusser Anwaltssozietät

(56) References cited:
- EP-A- 0 003 911
- FR-A- 2 089 624
- FR-A- 2 315 864
- GB-A- 1 506 052
- GB-A- 2 185 674
- E. SPREER: "Technologie der Milchverarbeitung", 3th edition, 1974, pages 355,356,91, VEB Fachbuchverlag, Leipzig, DE
- G. PENSO: "Index Plantarum Medicinalium Totius Mundi Eorumque Synonymorum",1984, page 294, O.E.M.F., Milano, IT
- COMMONWEALTH AGRICULTURAL BUREAU, abstract no. 0206272, 7C010-01239, 0C054-06355; 7W010-01505; L.-H.C. LEN: "Tissue culture of Culculigo latifolia Dry. exW.T.Ait. (Hypoxidaceae)", & GARDENS' BULLETIN, 1981, 34(2),203-208
- CHEMICAL ABSTRACTS, vol. 107, 1987, page 455, abstract no. 194889t, Columbus,Ohio, US; J. XU et al.: "Chemical constituents of Xianmao (Curculigoorchioides). I. Isolation and characterization of curculigine A",& ZHONGCAOYAO 1987, 18(5), 194-5, 222

## Description

### BACKGROUND OF THE INVENTION

This invention relates to a method for stabilizing a taste-modifier which comprises a curculin-containing material.

Known taste-modifiers which affect the receptor membranes on the tongue in such a manner as to modify the taste of a food, include those which remove the sweetness of a sweet food in the mouth, for example, gymnemic acid contained in Gymnema sylvestre leaves and ziziphine contained in Ziziphus jujuba leaves; and those which convert the sourness of a sour food into sweetness in the mouth, for example, miraculin contained in Synsepulm dulcificum fruits.

Although miraculin has the abovementioned effect, it is not put into practical use as a taste-modifier because of its poor stability.

The present inventors have found that a sour material or water taken after eating Curculigo latifolia fruits would taste sweet. Thus they have attempted to identify the sweetness-inducer. As a result, they have found that a specific protein contained in Curculigo latifolia fruits is the aimed sweetness-inducer (cf. Japanese Patent Application No. 153143/1988). This protein is named curculin. In order to utilize this curculin as a taste-modifier on a commercial scale, it is required to obtain crude curculin in a stable form from Curculigo latifolia fruits as efficiently as possible.

Pure curculin may be obtained by washing fresh Curculigo latifolia fruits or dried fruits thereof with water, extracting from them with an aqueous solution of a salt and purifying the extract by ion-exchange chromatography with the use of CM-Sepharose and HPLC with the use of a gel column. Pure or almost pure curculin would remain stable for a month or longer in the form of an aqueous solution at room temperature. However fresh Curuculigo latifolia fruits, dried fruits thereof or crude curculin, in particular, in the form of an aqueous solution, would have a poor stability upon storage since they are contaminated with proteases and bacteria contained in the Curculigo latifolia fruits.

### SUMMARY OF THE INVENTION

It is an object of the present invention to provide a method for stabilizing a taste-modifier comprising a protein curculin in a state of a crude product or an aqueous solution for a prolonged period of time.

The present inventors have conducted extensive studies in order to achieve the above object. As a result, they have found that the protein curculin is stable to heat and thus the taste-modification activity thereof would be never lowered when a curculin-containing material is heated in order to pasteurize the same as well as to inactivate proteases contained therein.

Accordingly the present invention, which has been completed based on the above finding, provides a method for stabilizing a taste-modifier comprising a curculin-containing material which comprises a) extracting a curculin-containing material from fresh Curculigo latifolia fruits or dried fruits thereof and b) subjecting said curculin-containing material to ultrahigh-temperature short-time pasteurization at 110 to 150°C for two seconds to two minutes.

According to the method for stabilizing a taste-modifier of the present invention, a taste-modifier comprising a curculin-containing material can be pasteurized and proteases contained therein can be inactivated without lowering the taste-modification activity of the same. Thus the taste-modifier can be stably preserved for a prolonged period of time in the form of a crude product or an aqueous solution.

### DETAILED DESCRIPTION OF THE INVENTION

The invention, which comprises subjecting the taste-modifier to ultrahigh-temperature short-time pasteurization at 110 to 150°C for two seconds to two minutes, is preferred in order to stabilize the taste-modifier in the form of a solution or a fluid, since the pasteurization and the inactivation of the protease can be completely effected and the tate-modifier can be filled under aseptic conditions.

The ultrahigh-temperature short-time pasteurization may be carried out with the use of and device, for example, a direct-heating one such as VTIS pasteurizer mfd. by Alfa-Laval or an indirect heating one such as a contherm scratching one. The taste-modification activity of the taste-modifier might be lowered when heated at 90°C or above for a long period. Thus the ultrahigh-temperature short-time pasteurization may be appropriately effected for two seconds to two minutes.

According to the present invention, the taste-modifier comprising a curculin-containing material is subjected to ultrahigh-temperature short-time pasteurization at 110 to 150°C for two seconds to two minutes to thereby stabilize said taste-modifier.

The method for stabilizing the taste-modifier of the present invention may be conducted at any stage in the purification of curculin. In addition, this method may be applied to the taste-modifier in any form, i.e., a dried matter, a solution or dispersion in water or a product containing the same.

Examples of the curculin-containing material obtained from fresh Curculigo latifolia fruits or dried fruits thereof described above include curculin extracted from fresh Curculigo latifolia fruits, dried fruits thereof or the residue obtained by appropriately treating the fresh Curculigo latifolia fruits or dried fruits thereof and removing a curculin-free component therefrom. The concentration of the curculin extracted from fresh Curculigo latifolia fruits or dried fruits thereof is not particularly restricted. Namely, either a highly pure curculin or an extract containing a large amount of materials other than the curculin may be used in the present invention. Further the extract may be mixed with other components.

The extraction of the curculin is not particularly restricted. A preferable example thereof comprises extracting from fresh Curculigo latifolia fruits or dried fruits thereof with an aqueous solution of a salt at a concentration of at least 0.01 M. Examples of the salt include chlorides such as sodium chloride, potassium chloride, calcium chloride, magnesium chloride and ammonium chloride; phosphates such as sodium phosphate, potassium phosphate, magnesium phosphate and ammonium phosphate; carbonates such as sodium carbonate, potassium carbonate, magnesium carbonate and ammonium carbonate; sulfates such as sodium sulfates, magnesium sulfate, calcium sulfate and ammonium sulfate; sulfites such as sodium sulfite, magnesium sulfite, calcium sulfite and ammonium sulfite; nitrates such as sodium nitrate and potassium nitrate; nitrites such as sodium nitrite and potassium nitrite; lactates such as sodium lactate and calcium lactate; alum; burnt alum; sodium acetate; pyrophosphates such as sodium pyrophosphate and potassium pyrophosphate; propionates such as sodium propionate and calcium propionate; sodium benzoate; sodium fumarate; and sodium polyacrylate.

A typical example of the extraction of curculin with the aqueous solution of a salt may be carried in the following manner.

An aqueous solution of a salt such as sodium chloride is added to fresh Curculigo latifolia fruits or dried fruits thereof and the obtained mixture is homogenized followed by filtering and centrifuging. Since curculin is contained in the water-insoluble part of Curculigo latifolia sarcocarp, it is preferable to homogenize the above mixture of the fresh Curculigo latifolia fruits or dried fruits thereof and water followed by thoroughly washing the mixture to thereby remove the water-soluble part and extracting from the residue with the above-mentioned salt solution so as to elevate the purity of curculin.

The concentration of the salt of the aqueous solution to be used for the extraction should exceed 0.01 M, since curuculin can not be sufficiently extracted with a salt solution of a concentration lower than 0.01 M. On the other hand, a salt solution of an excessively high concentration requires a prolonged period of time for desalting following the extraction. Thus the concentration of the salt solution preferably ranges from 0.1 to 1.0 M, from the viewpoints of the extraction efficiency and the subsequent purification procedure.

The extract thus obtained with the use of the salt solution is then desalted and dried to thereby give a curculin-containing material which is sufficiently available in practice. However the purity of curculin can be further elevated by purifying the above extract by ion exchange chromatography with the use of CM-Sepharose and HPLC with the use of a gel column followed by desalting and drying. Thus pure curculin can be obtained. It is a matter of course that the curculin purity may be further elevated by various purification procedures other than those described above, for example, known protein purification procedures such as salting-out or solvent precipitation.

A typical example of the curculin thus obtained is a protein having a molecular weight of approximately 12.500 dalton, an amino acid residue number of 97 and an iso-electric point of 7.1. This protein is present as a dimer of a molecular weight of approximately 26.000 dalton. The following Table 1 shows the amino acid composition of this protein. Thus it contains relatively large amounts of aspartic acid, leucine and glycine.

**Table 1**

| Amino acid composition | | |
|---|---|---|
| Amino acid | % by mol | No. of residues |
| Aspartic acid (Asp) | 17.3 | 17 |
| Threonine (Thr) | 6.4 | 6 |
| Serine (Ser) | 7.0 | 7 |
| Glutamic acid (Glu) | 7.2 | 7 |
| Proline (Pro) | 1.2 | 1 |
| Glycine (Gly) | 12.5 | 12 |
| Alanine (Ala) | 5.3 | 5 |
| Cystine (Half-cys) | - | - |
| Valine (Val) | 6.8 | 7 |
| Methionine (Met) | 0.4 | 1 |
| Isoleucine (Ile) | 4.2 | 4 |
| Leucine (Leu) | 14.5 | 14 |
| Tyrosine (Tyr) | 5.2 | 5 |
| Phenylalanine (Phe) | 1.3 | 1 |
| Lysine (Lys) | 2.7 | 3 |
| Histidine (His) | 2.4 | 2 |
| Arginine (Arg) | 5.5 | 5 |
| Total | | 97 |

To further illustrate the present invention, the following Examples will be given.

### Example 1

100 1 of water was added to 20 kg of Curculigo latifolia sarcocarp ground in a mortar. Then the mixture was homogenized and centrifuged at 10.000 rpm for 30 minutes. After removing the supernatant, 100 1 of water was added to the residue and the obtained mixture was homogenized and centrifuged, followed by removing the supernatant. To the obtained residue 30 1 of a 0.5 M NaCl solution was added and the mixture was homogenized in a mixer for two minutes and then filtered under reduced pressure. After collecting the filtrate, 30 1 of a 0.5 M NaCl solution was further added to the residue. The mixture was homogenized and filtered under reduced pressure followed by collecting the filtrate.

These filtrates were combined and centrifuged at 30.000 rpm for one hour to thereby give a crude curculin extract as the supernatant.

This crude extract was desalted by ultrafiltration and concentrated to a volume of 5 1. Then it was pasteurized with a VTIS pasteurizer (mfd. by Alfa-Laval) at 130°C for five seconds. Then it was aseptically filled in a container to thereby give a taste-modifier in the form of an aqueous solution.

1 ml of this taste-modifier aqueous solution was kept in the mouth for a minutes and then vomitted. A 0.02 M aqueous solution of citric acid, which was taken thereafter, tasted highly sweet just like a sugar solution does.

The container was allowed to stand as such for three months and then opened. Then the content was subjected to the same sensory test as the one described above. As a result, it tasted highly sweet similar to the above case.

### Comparative Example 1

Curculigo latifolia sarcocarp ground in the same manner as the one described in Example 1 was lyophilized to thereby give a taste-modifier.

Immediately after the preparation, this taste-modifier showed a taste-modification effect comparable to that of the taste-modifier of Example 1. After allowing to stand in a sterile Petri dish for three months at 37°C, however, it showed no sweetness but a sourness in the same sensory test as the one described above.

### Comparative Example 2

50 ml of water was added to 10 g of Curculigo latifolia sarcocarp ground in the same manner as the one described above. Then the mixture was homogenized and centrifuged at 10.000 rpm for 30 minutes. After removing the supernatant, 50 ml of water was added to the resuide and the obtained mixture was homogenized. Then it was centrifuged at 10,000 rpm for 30 minutes. After removing the supernatant, the residue was obtained.

This residue was dried under reduced pressure to thereby give a taste-modifier.

10 mg of this taste-modifier was kept in the mouth for a minutes and then vomitted. Subsequently a 0.02 M aqueous solution of citric acid was taken. As a result, the citric acid solution showed an intense sweetness just like a sugar solution does.

This taste-modifier was introduced into a sterile Petri dish and allowed to stand at 37°C for three months. Then it was subjected to the same sensory test as the one described above. As a result, it showed a slight sweetness and an intense sourness.

## Claims

1. A method for stabilizing the taste-modifying properties of a curculin-containing material which comprises:
a) extracting a curculin-containing material from fresh Curculigo latifolia fruits or dried fruits thereof and
b) subjecting said curculin-containing material to ultrahigh-temperature short-time pasteurization at 110 to 150°C for two seconds to two minutes.

## Patentansprüche

1. Verfahren zum Stabilisieren der geschmacksverwandelnden Eigenschaften eines Curculin-enthaltenden Materials, welches umfaßt:
a) Extrahieren eines Curculin-enthaltenden Materials aus frischen oder getrockneten Früchten von Curculigo latifolia und
b) Aussetzen des besagten Curculin-enthaltenden Materials einer Ultrahochtemperatur-Kurzzeit-Pasteurisierung bei 110 bis 150° C für zwei Sekunden bis zwei Minuten.

## Revendications

1. Procédé pour stabiliser les propriétés de modification du goût d'une matière contenant de la curculine, dans lequel:
a) on extrait une matière contenant de la curculine à partir de fruits frais ou de fruits séchés de Curculigo latifolia, et
b) on soumet ladite matière contenant de la curculine à une pasteurisation de courte durée à très haute température à 110 à 150°C pendant deux secondes à deux minutes.
